# EUROPÄISCHE PATENTSCHRIFT

(11) **EP 1 065 271 B1**
(45) Veröffentlichungstag und Bekanntmachung des Hinweises auf die Patenterteilung: **25.03.2009**
(21) Anmeldenummer: 00118282.3
(22) Anmeldetag: 06.07.1992
(51) Int. Cl.: C12N 15/13, C12N 15/10, C12P 21/08, C12Q 1/68, C07K 16/00, G01N 33/53

(54) **Phagemid zum Screenen von Antikörpern**
Phagemide for screening antibodies
Phagemide utilé pour trier des anticorps

(30) Priorität: 08.07.1991 DE 4122599
(43) Veröffentlichungstag der Anmeldung: 03.01.2001
(62) Teilanmeldung aus: 92914199.2
(73) Patentinhaber: Deutsches Krebsforschungszentrum Stiftung des öffentlichen Rechts, 69120 Heidelberg (DE)
(72) Erfinder: Breitling, Frank, Dr., 69120 Heidelberg (DE); Little, Melvyn, Prof.Dr., 69151 Neckargemünd (DE); Dübel, Stefan, Dr., 69221 Dossenheim (DE); Braunagel, Michael, Dr., 0383 Oslo (NO); Klewinghaus, Iris, 68165 Mannheim (DE)
(74) Vertreter: Schüssler, Andrea

(56) Entgegenhaltungen:
- WO-A-90/02809
- WO-A-91/17271
- WO-A-91/18980
- WO-A-92/01047
- WO-A-92/09690
- MCCAFFERTY J ET AL: "PHASE ANTIBODIES: FILAMENTOUS PHAGE DISPLAYING ANTIBODY VARIABLE DOMAINS" NATURE,GB,MACMILLAN JOURNALS LTD. LONDON, Bd. 348, 6. Dezember 1990 (1990-12-06), Seiten 552-554, XP002024992 ISSN: 0028-0836
- PARMLEY S F ET AL: "ANTIBODY-SELECTABLE FILAMENTOUS FD PHAGE VECTORS: AFFINITY PURIFICATION OF TARGET GENES" GENE,NL,ELSEVIER BIOMEDICAL PRESS. AMSTERDAM, Bd. 73, Nr. 2, 20. Dezember 1988 (1988-12-20), Seiten 305-318, XP002072154 ISSN: 0378-1119
- WARD E S ET AL: "BINDING ACTIVITIES OF A REOPERTOIRE OF SINGLE IMMUNOGLOBULIN VARIABLE DOMAINS SECRETED FROM ESCHERICHIA COLI" NATURE,GB,MACMILLAN JOURNALS LTD. LONDON, Bd. 341, Nr. 6242, 12. Oktober 1989 (1989-10-12), Seiten 544-546, XP000086104 ISSN: 0028-0836
- CWIRLA S E ET AL: "PEPTIDES ON PHAGE: A VAST LIBRARY OF PEPTIDES FOR IDENTIFYING LIGANDS" PROCEEDINGS OF THE NATIONAL ACADEMY OF SCIENCES OF USA,US,NATIONAL ACADEMY OF SCIENCE. WASHINGTON, Bd. 87, Nr. 16, 1. August 1990 (1990-08-01), Seiten 6378-6382, XP000141872 ISSN: 0027-8424
- SCOTT J K ET AL: "SEARCHING FOR PEPTIDE LIGANDS WITH AN EPITOPE LIBRARY" SCIENCE,US,AMERICAN ASSOCIATION FOR THE ADVANCEMENT OF SCIENCE,, Bd. 249, 27. Juli 1990 (1990-07-27), Seiten 386-390, XP002003037 ISSN: 0036-8075
- DEVLIN J J ET AL: "RANDOM PEPTIDE LIBRARIES: A SOURCE OF SPECIFIC PROTEIN BINDING MOLECULES" SCIENCE,US,AMERICAN ASSOCIATION FOR THE ADVANCEMENT OF SCIENCE,, Bd. 249, 27. Juli 1990 (1990-07-27), Seiten 404-406, XP000616029 ISSN: 0036-8075
- BREITLING F ET AL: "A SURFACE EXPRESSION VECTOR FOR ANTIBODY SCREENING" GENE, Bd. 104, 15. August 1991 (1991-08-15), Seiten 147-153, XP002046118 Elsevier Science Publishers B.V., NL

## Beschreibung

Die vorliegende Erfindung betrifft Phagemide zur Selektion von spezifischen, aus großen Genbanken erhaltenen Antikörpern, die Herstellung dieser Phagemide und ihre Verwendung zur Selektion von spezifischen, aus großen Genbanken erhaltenen Antikörpern unter Verwendung geringer Mengen von Antigen.

Plasmid- und Phagen-Antikörper-Genbanken wurden in E.coli von PCR-amplifizierten Immunglobulin-Familien nach Immunisierung entwickelt. Rekombinante Antikörper gegen Immunogene wurden durch ELISA-Tests der Bakterienüberstände von isolierten Bakterienkolonien (Ward, E.S., Güssow, D., Griffiths, A.D., Jones, P.T. and Winter, G., "Binding activities of a repertoire of single immunoglobulin variable domains secreted from Escherichia coli", Nature (1989), 341, 544-546) oder durch Screenen von auf Nitrocellulose übertragenen Plaques von Bakterienkolonien auf Reaktivität gegen radioaktiv markierte Immunogene (Huse, W.D., Sastry, L., Iverson, S.A., Kang, A.S., Alting-Mees, M., Burton, D.R., Benkovic, S.J. and Lerner, R.A., "Generation of a large combinatorial library of the immunoglobin repertoire in phage lambda," Science (1989), 246, 1275-1281) selektiert. Zur Selektion jedoch von spezifischen Antikörpern von Genbanken willkürlich kombinierter leichter und schwerer Ketten von nicht-immunisierten Tieren, bei denen kein Übergewicht von Antikörpern für ein betimmtes Antigen vorliegt, ist ein Verfahren zum Screenen von Millionen von Antikörper-produzierenden Bakterien notwendig.

Ein möglicher Weg, einen großen Bereich von Antikörpern zu screenen, liegt darin, rekombinante Antikörper an die Oberfläche von Bakterien oder Bakteriophagen zu binden, sodaß sie dann rasch durch an eine feste Phase gebundene Antigene selektiert werden können. Hinsichtlich der Schwierigkeiten, Proteine gezielt auf die Zelloberfläche von Bakterien zu bringen, ist die M13-Familie von filamentösen Bakteriophagen wegen ihrer kleinen Größe und ihres relativ einfachen genetischen Aufbaus ein verlockender Kandidat (vgl. Übersichtsartikel von Webster, R.E. and Lopez, J., in "Virus Structure and Assembly", herausgegeben von S. Casjens, veröffentlicht von Jones and Bartlett Inc., Bosten/Portala Valley, USA, 1985; Day, L.A., Marzec, C.J., Reisberg, S.A. and Casadevall, A., "DNA packaging in filamentous bacteriophages", Ann. Rev. Biophys. Chem. (1988), 17, 509-539).

Das Produkt von Gen III (pIII) ist ein relativ flexibles und zugängliches Molekül, das aus zwei funktionellen Domänen zusammengesetzt ist; einer amino-terminalen Domäne, die an den F-Pili männlicher Bakterien während der Infektion bindet, und einer carboxy-terminalen, innerhalb des Virion verborgenen Domäne, die für die Morphogenese wichtig ist. Peptide können zwischen den zwei Domänen von pIII (Smith, G.P., "Filamentous fusion phage: novel expression vectors that display cloned antigens on the virion surface", Science (1985), 228, 1315-1317) oder in der Nähe des N-Terminus (Parmley, S.F. and Smith, G.P., "Antibody-selectable filamentous fd phage vectors: affinity purification of target genes", Gene (1988), 73, 305-318) ohne Zerstörung seiner Funktionen in Morphogenese und Infektion inseriert werden. Nach erheblicher Pionierarbeit über die Verwendung von pIII in fd Phagen zum Führen fremder Peptide, zeigten Parmely and Smith (1988, vorstehend angegeben), daß Peptidepitope, die am amino-terminalen Ende inseriert sind, Phagen an immobilisierte Antikörper binden können. In Folge dieser Arbeit war es möglich, Peptid-Genbanken zu entwickeln, die man auf Bindung an Liganden und Antikörpern screenen kann (Scott, J.K. and Smith, G.P. "Searching for peptide ligands with an epitope library", Science (1990), 249, 386-390; Devlin, J.J., Panganiban, L.C. and Devlin, P.E., "Random peptide libraries: A source of specific protein binding molecules", Science (1990), 249, 404-406; Cwirla, S.E., Peters, E.A., Barrett, R.W. and Dower, W.J., "Peptides on phage, a vast library of peptides for identifying ligands", Proc. Natl. Acad. Sci. USA (1990),87, 6378-6382).

McCafferty, J., Griffith, A.D., Winter, G. and Chiswell, D.J., "Phage antibodies: filamentous phage displaying antibody variable domains", Nature (1990), 348, 552-554 berichteten über den Zusammenbau eines Antikörper-pIII-Fusionsproteins in einen fd-Phagen mit einem Tet^{R}-Gen nach Insertion der Antikörper-DNA in das 5'-Ende von Gen III. Der Phage blieb infektiös und konnte durch Affinitäts-Chromatographie angereichert werden. Die Fusionsphagen zeigten sich jedoch hauptsächlich für relativ kleine Inserts als geeignet, wahrscheinlich weil die großen Inserts einen negativen Einfluß auf die Infektivität von pIII haben (Parmlee and Smith, 1988, vorstehend angegeben). Daher besteht ein großes Risiko, daß in Phagen-Genbanken nach ihrer Amplifikation schnell Deletionsmutanten dominieren.

Die WO92/01047, die am 23. Januar 1992 veröffentlicht wurde und ein Dokument nach Artikel 54(3) EPÜ ist, offenbart spezifische Phagemidkonstrukte, die Fusionsproteine von spezifischen Antikörperfragmenten am N-Ende des pIII Proteins vom fd Phagen kodieren. Diese Phagemide basieren auf dem Vektor pUC119. Die spezifisch beschriebenen Phagemide werden als pCAT-3scFvD1.3 und die dazu eng verwandte Gruppe der pHEN1 I-IV Phagemide bezeichnet. In pCAT-3scFvD1.3 ist das kodierte Antikörperfragment ein Einzelketten Fv eines Antikörpers, D1.3, gegen das Lysozym aus dem Hühnerei. Die das Antikörperfragment kodierende Nukleinsäuresequenz, die in Fig. 5 der WO 92/01047 dargestellt ist, und das das Antikörper pIII Protein kodierende Konstrukt werden in den pUC 119 geklont. In den Phagemiden pHEN1 I-IV kodieren die Konstrukte die variablen Regionen V_{H} der schweren und/oder V_{L} der leichten Kette des Mausantikörpers NQ10.12.5 zusammen mit der Ck und CH1 (Gamma1 Isotyp) gegen 2-Phenyl-5-Oxazolon. In der WO92/01047 wird auf Griffiths et al., Nature 312, 271-275, 1984 verwiesen, worin der NQ10.12.5 Antikörper offenbart wird; Griffiths *et al*. stellen die Nukleinsäuresequenzen der Antikörpergene bereit.

Die vorliegende Erfindung betrifft die Verwendung eines Phagemids umfassend DNA kodierend für ein Fusionsprotein aus einem Antikörper und einem Coliphagen pIII-Protein wobei das Coliphagen plll-Protein die Amino-terminale Domäne und die Carboxy-terminale Domäne eines Coliphagen pIII-Proteins umfasst, in einer substraktiven Selections-Methode unter Verwendung von normalen und neoplastischen Zeilen zur Identifizierung von Tumor-assoziierten Antigenen.

Vorzugsweise ist der Antikörper ein Einzelketten-Antikörper.

DNA, die für ein Antikörper-pIII-Fusionsprotein, vorzugsweise ein Einzelketten-Antikörper-pIII-Fusionsprotein, codiert, wurde in ein Phagemid eingebaut. Ein großer Vorteil des erfindungsgemäßen Phagemid-Systems gegenüber McCafferty et al. (vorstehend angegeben) liegt darin, daß es als Plasmid vermehrt werden kann und nicht unter Selektionsdruck bezüglich der Entfernung von Antikörper-DNA steht, da die Expression des Fusionsproteins stark unterdrückt ist. Dies ist insbesondere während der Amplifikation der Antikörper-Genbanken wichtig, wenn schneller proliferierende Deletionsmutanten rasch dominant werden könnten. Die Phagemid DNA, die weniger als die Hälfte obiger Phagen DNA ausmacht, transformiert auch Bakterien effizienter. Darüberhinaus werden im Gegensatz zu obigem Phagensystem große Mengen der kleineren Phagemid DNA produziert und große Mengen von Antikörperprotein sind nach Induktion verfügbar, wodurch die Analyse stark erleichtert wird.

Die Expression des Antikörper-pIII-Fusionsproteins, insbesondere des Einzelketten-Antikörper-pIII-Fusionsproteins, unter Verwendung des pSEX-Phagemids und seiner Verpackung in virale Partikel erleichtert die Entwicklung bakterieller Systeme zur Isolierung von Antikörpern hoher Affinität. Millionen Antikörper-produzierende Klone von Antiköper-Genbanken kann man jetzt rasch durch Bindung an immobilisierte Antigene screenen. Ein weiterer Vorteil gegenüber herkömmlichen Verfahren zum Screenen liegt darin, daß nur kleine Mengen Antigen benötigt werden, ein wichtiger Faktor, wenn der Vorrat eines seltenen Proteins begrenzt ist. Dieses System ermöglicht auch, zufällig mutierte Antikörper zu screenen, um ihre Bindungs-Affinitäten zu erhöhen. Das Verfahren kann mehrfach wiederholt werden, bis die gewünschte Spezifität erreicht ist. Es ist nun zum ersten Mal möglich, in großem Maßstab unterschiedliche Tests zum Screenen von verwandten Zellen und Organismen durchzuführen. Eine subtraktive Selektion, beispielsweise unter Verwendung von normalen und neoplastischen Zellen, kann zur Identifizierung von Tumor-assoziierten Antigenen verwendet werden. Das Phagemid-System erweist sich auch als äußerst hilfreich für die Untersuchung molekularer Wechselwirkungen, beispielsweise durch Selektion von Antikörpern, die eine Liganden-Rezeptor-Bindung verhindern.

Die folgenden Beispiele erläutern die Erfindung.

### Beispiel 1

### Konstruktion eines Phagemids (pSEX)

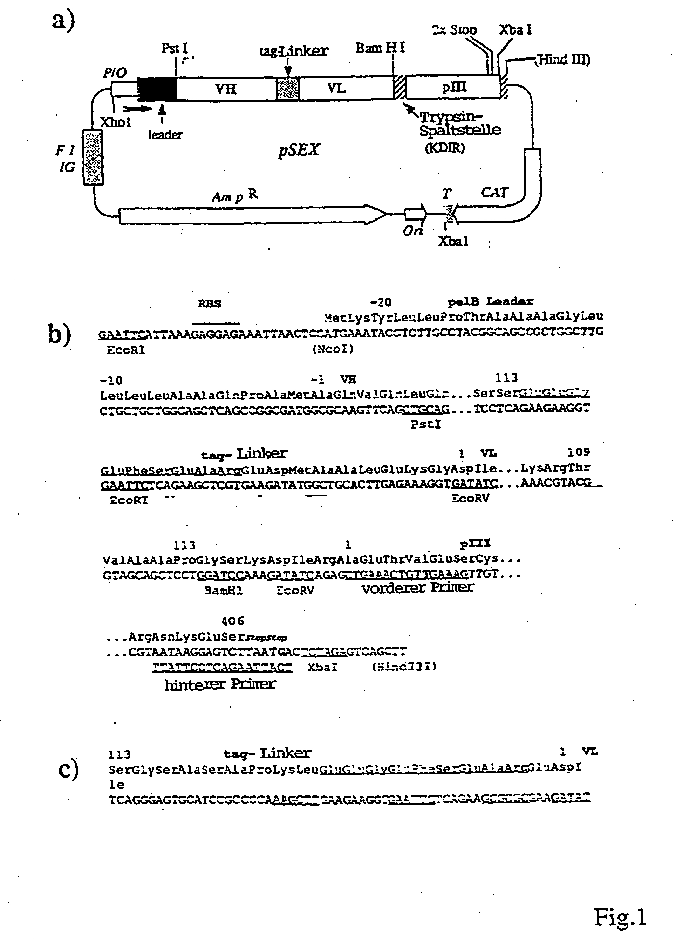

DNAs, die für einen Einzelketten-Antikörper (scAb) und pIII codieren, wurden nach Insertion eines spezifischen Satzes von Restriktionsstellen und einer Protease-sensitiven Verbindungssequenz in die multiple Klonierungsstelle in pUC 119 kloniert. Die Ab-DNA codierte für die variablen Domänen der schweren und leichten Kette eines humanisierten Ab gegen Hühnereiweiß-Lysozym, der von dem Anti-Lysozym Ab D1.3 stammte (Amit et al., Science (1986), 233, 747-754; Verhoeyen, M. et al., Science (1988), 239, 1534-1536). Diese Domänen wurden über eine 18-Aminosäure-Linker-Sequenz verbunden, die das Epitop des monoklonalen Ab YOL1/34 enthielt (Breitling, F. and Little, M., "Carboxy-terminal regions on the surface of tubulin and microtubules: Epitope locations of YOL1/34, DM1A and DM1B", J. Mol. Biol. (1986), 189, 367-370), wodurch der Ab identifiziert werden konnte. Zur Bereitstellung einer flexibleren Verbindung zu pIII, wurde das 3'-Ende der DNA für die leichte Kette durch Zusatz von Nucleotiden, die für die ersten sechs Aminosäuren der konstanten Domäne in der menschlichen Kappa-Kette codierten, gefolgt von einer BamHI-Restrictionsstelle modifiziert. pIII DNA wurde von dem Bakteriophagen M13 amplifiziert, indem Primer entsprechend der 5'- und 3'-Enden des Gens III verwendet wurden. Die Ab-pIII-DNA wurde dann in einem Phagemid der pDS-Familie kloniert, das einen mit zwei lac-Operatoren kombinierten Coliphagen-T7-Promotor enthielt (Bujard, H., Gentz, R., Lancer, M., Stüber, D., Müller, H.-M., Ibrahim, I., Häuptle, M-T. and Dobberstein, B., "A T5 promoter-based transcription-translation system for the analysis of proteins in vitro and in vivo", Methods Enzymol. (1987), 155, 416-433; Lancer, M. and Bujard, H., "Promoters determine the efficiency of repressor action", Proc. Natl. Acad. Sci. USA (1988), 85, 8973-8977; Müller, H.-M., Ph. D. thesis, Univ. Heidelberg, 1989). Schließlich wurde eine für die Leader-Sequenz des bakteriellen Enzyms Pektatlyase codierende DNA an das 5'-Ende der Ab-DNA ligiert, wodurch das Phagemid pSEX (Fig. 1a) erhalten wurde. Die Leader-, Linker- und PCR-Primer-Sequenzen sind in Figur 1b gezeigt. Eine alternative Linker-Sequenz (Fig. 1c) mit dem YOL1/34 Epitop am Ende des Linkers, die eine nützliche Restriktionsstelle zur Insertion von Ab-Genbanken enthielt, wurde auch verwendet. Obwohl beide tag-Linker eine bedeutsame Menge saurer Reste enthielten, zeigten sie sich ohne Effekt auf die Herstellung von funktionellen scAbs im Vergleich mit scAbs, deren Linker nur aus den neutralen Aminosäuren Glycin und Serin zusammengesetzt waren.

### Beispiel 2

### Expression eines Antikörper-pIII-Fusionsproteins

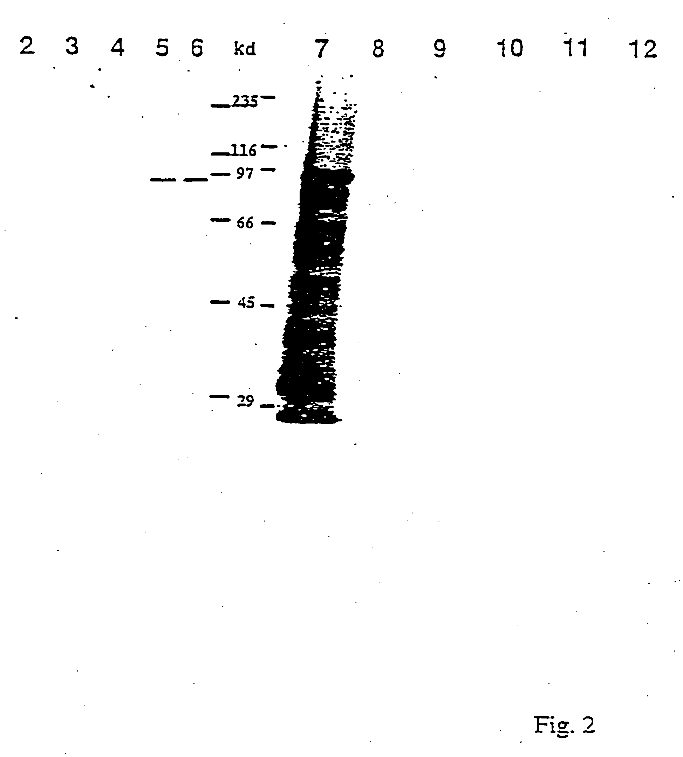

Zur Überprüfung, ob der fertige Phagemid-Vektor in der Lage war, das Fusionsprotein in voller Länge zu exprimieren, wurden 100 µm IPTG einer sich in log-Phase befindlichen, mit pSEX transformierten E. coli-Kultur zugegeben. Die Kultur zeigte einen deutlichen Rückgang in ihrer Wachstumsrate, verglichen mit der Kontrolle, was auf eine bedeutsame Synthese eines Phagemid-codierten Proteins hinwies. In der Western-Blot-Analyse wurde das Antikörper-pIII-Konstrukt durch drei Antikörper identifiziert; einem monoklonalen Antikörper gegen einen Teil der Linker-Sequenz (EEGEFSEAR) und zwei Anti-Peptid-Kaninchenseren gegen N-terminale Sequenzen der schweren und leichten Ketten (QVQLQQSGGG bzw. DJQMTQSPSS). Es wanderte mit einem offensichtlichen Molekulargewicht von 93 kd (Fig. 2). Die große Größe des Fusionsproteins (vorausgesagt: Mr 68.100) ist höchstwahrscheinlich durch die pIII-Komponente (Mr 42.100) bedingt, die mit einem offensichtlichen Molekulargewicht von etwa 55.000 - 70.000 kd wandert (Goldsmith, M.E. and Königsberg, W.H., "Adsorption protein of the bacteriophage fd: isolation, molecular properties and location in the virus", Biochemistry (1977), 16, 2868-2694). Partielle Proteolyse des Fusionsprotein wurde durch das Vorliegen einiger schwächerer Banden niedrigeren Molekulargewichts angezeigt, die mit den drei Antikörpern identisch angefärbt wurden.

Eine Zellfraktionierung zeigte, daß das Protein in den Cytoplasma und Membran-Fraktionen vorlag, nicht aber im Periplasma und dem Kulturüberstand (Fig. 2, Spuren 3-6) im Gegensatz zu der Antikörper-Komponente allein ohne pIII, die in das Periplasma und das Medium sekretiert wurde (Daten nicht gezeigt). Dies war nicht überraschend, da pIII auf Phagenpartikeln aus der inneren bakteriellen Membran zusammengesetzt wird, ein Prozeß, der scheinbar nur von der C-terminalen Domäne abhängt. Deletionsmutanten von pIII ohne diese Domäne gelangen in das Periplasma, ohne an die cytoplasmatische Membran gebunden zu werden (Boeke, J.D. and Model, P., "A prokaryotic membrane anchor sequence: carboxyl terminuns of bacteriophage fl gene III protein retained in the membrane", Proc. Natl. Acad. Sci. USA (1982), 79, 5200-5204), und normale Phagenpartikel werden nicht zusammengesetzt (Crissman, J.W. and Smith, G.P., "Gene III protein of filamentous phages: evidence for a carboxy-terminal domain with a role in morphogenesis", Virology (1984), 132, 445-455). Die Anker-Sequenz ist wahrscheinlich eine hydrophobe Strecke von 23 Aminosäuren am Carboxy-Terminus (Davis, N.G., Boeke, S. and Model, P., "Fine structure of a membrane anchor domain", J. Mol. Biol. (1985), 181, 111-121).

Die Fähigkeit des Fusionsproteins, ein Antigen zu binden, wurde untersucht, indem die Triton-lösliche Fraktion über eine an Sepharose gebundene Lysozymsäule gegeben wurde. Western Blots des ungebundenen Materials und der nach gründlichem Waschen und Eluieren mit 0,05 M Dimethylamin erhaltenen Fraktionen zeigten, daß das Fusionsprotein in voller Länge tatsächlich spezifisch auf der Lysozymsäule zurückgehalten wurde (Fig. 2, Spuren 7-12).

### Beispiel 3

### Verpackung des pSEX-Phagemids

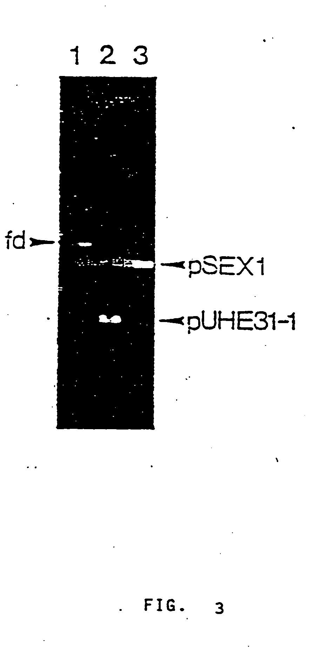

Zur Bestimmung, ob der Phagemid-Expressionsvektor verpackt werden konnte, wurden pSEX-enthaltende E.coli mehrfach mit dem Phagen fd infiziert. IPTG wurde nicht zugegeben, da gefunden wurde, daß es einen inhibitorischen Effekt auf das Verpacken eines Phagemids hat. Ähnliches wurde kürzlich von Bass et al., Proteins (1990), 8, 309-314 berichtet, der ein Phagemid konstruierte, das ein Fusionsprotein aus humanem Wachstumshormon und der C-terminalen Domäne von pIII exprimierte. Die Untersuchung der Ab-pIII-Produktion mit und ohne IPTG nach Zugabe des Phagen fd zeigte, daß der Phage allein in der Lage war, eine Expression zu induzieren (Fig 3). Eine mögliche Erklärung hierfür ist, daß eines der Phagen-Gen-Produkte die Bindung des lac-Repressors an den Operator stört. Andererseits könnte die Bindung von Phagen-Proteinen an die Zwischengenregion die Topologie des Phagemid beeinflußen und die Freisetzung des lac-Repressors bewirken. Wie auch immer, wir haben gefunden, daß das Durchwandern der Zwischengenregion 10³ Nukleotide zu der anderen Seite des bla-Gens keinen Effekt auf dieses Phänomen hat (Daten nicht gezeigt).

Agarose-Gelelektrophorese der von Viruspartikeln in das Medium abgegebenen DNA zeigt zusätzlich zu der Einzelstrang-DNA von fd eine größere Menge kleinerer DNA, die in der Größe mit einzelsträngigem pSEX vergleichbar war. Ein weiterer Beweis für eine Phagemid-Verpackung und der Produktion von infektiösen Partikeln wurde durch Infektion von E.coli mit den abgegebenen Virus-Partikeln erbracht. 10¹⁰ ml Amp^{R} E.coli Kolonien wurden im Vergleich zu 3x10⁹ pfu erhalten.

Zur Bestimmung, ob das verpackte Phagemid das Antikörper-pIII-Fusionsprotein eingebaut hatte, wurden 90 µl Kulturüberstand, der 5x10⁸ verpackte, als Amp^{R} transduzierende Einheiten bestimmte Phagemide enthielt, mit einem tausend-fachen Überschuß an Wildtyp fd-Phage gemischt, und über eine Säule immobilisierten Lysozyms gegeben. Nach gründlichem Waschen mit 10-fachem Badvolumen von PBS, 1 M NaCl bzw. 0,5 M NaCl in 0,1 M NaHCO₃ bei pH 8,3 wurden die Phagemid-Partikel mit 0,05 M Diethylamin eluiert. Das Eluat wurde mit 0,5 M NaH₂PO₄ neutralisiert und auf die Anzahl der Phagen und verpackten Phagemide getestet (Tabelle). Eine spezifische Anreicherung von bis zu 121-fach wurde erreicht, was den Einbau von funktionellen Antikörper-pIII-Konstrukten in die Phagemid-Partikel zeigte. Die Bindungseigenschaften der Phagemid-Partikel können weiter erhöht werden, indem eine pIII Deletionsmutante zum Verpacken verwendet wird. Dies garantiert, daß nur jene Phagemide, die für funktionelle Fusionsproteine codieren, verpackt werden und alle fünf pIII-Proteine auf einem Phagemid-Partikel mit Antikörper fusioniert werden.

### Beschreibung der Figuren

- Figur 1:: Konstruktion von pSEX, einem Phagemid zum Screenen von Antikörpern

VH und VL sind variable Domänen der schweren bzw. leichten Kette eines Anti-Lysozym-Ab.

### (a) Konstruktion

Zur Bereitstellung der notwendigen Restriktionsstellen, wurden die Oligonukleotide 5'GCTGAATTCGGATCCATAGGGCCCTCTAGAGTCGAC3' und 5' AATTGTCGACTCTAGAGGGCCCTATGGATCCGAATTCAGCTGCA3' 5'-phosphoryliert, miteinander hybridisiert und in pUC119 ligiert, der mit PstI und EcoR1 geschnitten und dephosphoryliert war. Gegebenenfalls wurden zur Schaffung einer Protease-sensitiven Sequenz die hybridisierten Oligonucleotide 5' GATCCAAAGATATCAGAGGGCC3' und 5'CTCTGATATCTTTG3' zwischen die BamHI- und ApaI-Stellen des ersten Oligonucleotid-Satzes inseriert. scAB-DNA wurde dann zwischen die PstI- und BamHI-Stellen inseriert, gefolgt von der Ligierung der pIII-DNA über stumpfe Enden nach Spaltung des Phagemids mit ApaI und Behandlung mit T4-DNA-Polymerase zur Entfernung 3'-überhängender Enden. pSEX wurde hergestellt, indem die multiple Klonierungsstelle von pUHE 24-2 mit dem nahe verwandten Phagemid pDS31-1 kombiniert wurde, das eine zusätzliche fl Zwischengenregion enthielt (Bujard et al., 1987, vorstehend angegeben; Müller, 1989, vorstehend angegeben). die pDS31-1-Sequenz erstreckt sich von XhoI entgegen des Uhrzeigersinns bis zu einer HindIII-Stelle (in Klammern), die nach Ligierung über stumpfe Enden verloren wurde. pUHE24-2 ist im wesentlichen mit pDSG identisch (Bujard et al., 1987), bei dem ein Choli-Phage T7-Promotor mit zwei lac-Operatoren und einer Ribosomen-Bindungsstelle kombiniert ist (PA1/04/03, Lancer and Bujard, 1988, vorstehend angegeben; Lanzer, 1988, vorstehend angegeben). Das erhaltene Phagemid wurde mit HindIII geschnitten und die 5'-überhängenden Enden wurden mit Klenow-Fragment aufgefüllt. Nach einer weiteren Spaltung mit PstI, wurde das PstI-HincII Ab-pIII-DNA-Fragment in das Phagemid inseriert. Schließlich wurde eine synthetische DNA, die für die Leader-Sequenz des Bakterienenzyms Pektatlyase und für die ersten vier Aminosäuren der schweren Kette codiert, zwischen die NcoI und PstI-Restriktionsstellen inseriert pUHE-Plasmide wurden in E.coli 71-18 mit dem Plasmid pDM1, das den lac-Repressor exprimiert, vermehrt, pUC-Plasmide wurden in DH5 vermehrt und das Antikörper-pIII-Fusionsprotein in JM101 exprimiert.

### (b) Sequenz der Ribosomenbindungsstelle (RBS), der Leader-Sequenz der Pektatlyase, des tag-Linkers und der PCR-Primer für pIIl.

Unterstrichene Aminosäuren geben das Epitop für YOL1/34 an. Die folgenden Aminosäuren in der Linker-Sequenz sind eine Fortführung der -Tubulin-Sequenz.

### (c) Mögliche tag-Linker-Sequenz.

Unterstrichene Aminosäuren geben das Epitop für YOL11/34 an. Die Aminosäuren des vorhergehenden Linkers sind eine Fortführung der Ab-Sequenz in die konstante Domäne.
- Fig. 2:: Induzierbarkeit, zelluläre Lokalisierung und Antigenbindung des Antikörper-pIII-Fusionsproteins, analysiert durch Gelelektrophorese auf 8 % Polyacrylamidgelen und Western Blott.

Spuren 1 und 2: Gesamte Zellen nach 1 h Inkubation mit 100 uM IPTG (1) oder ohne IPTG (2).

Spuren 3 - 6: Zellfraktionierung; 3: Kulturüberstand, 4: periplasmatisch angereicherte Fraktion, 5: lösliche cytoplasmatische Fraktionierung, 6: 1 % Tritonextrakt. Spuren 7 - 12: Lysozym-Affinitätschromatographie des 1 % Tritonextraktes von induzierten und nicht-induzierten Zellen, 7: Ausfluß (+IPTG), 8: Ausfluß (-IPTG), 9: letzte Waschung (+IPTG), 10: letzte Waschung (-IPTG), 11: Eluat (+IPTG), 12: Eluat (-IPTG).

Spuren 1 - 6 wurden angefärbt unter Verwendung des monoklonalen Antikörpers YOL1/34 (Serotec, Oxford, U.K.) und Spuren 7 - 12 unter Verwendung eines Antiserums gegen die N-terminale Sequenz der leichten Kette.

### Durchführung:

Antiseren gegen die schweren und leichten Ketten wurden durch subkutane Injektion von Kaninchen mit den amino-terminalen Peptiden QVQLQQSGGG(AC) bzw. DIQMTQSPSS(AC) erhalten, die an das Heamocyanin der Schlüsselloch-Napfschnecke gekoppelt waren. Zur Untersuchung der Expression des Fusionsproteins wurden pelletierte Bakterien von IPTG-induzierten Kulturen in 30 mM Tris/HCl, pH 8,0 Puffer resuspendiert, der 20 % Sucrose, 1 mM EDTA, 1 mg/ml Hühnerlysozym enthielt, und 10 min auf Eis inkubiert. Nach 1 minütiger Zentrifugation bei 15 000 g wurde der die periplasmatischen Proteine enthaltende Überstand gesammelt und das Pellet in 0,1 M Tris/HCl, pH 8,0 beschallt. Die lösliche cyctosolische Fraktion wurde nach 5 minütiger Zentrifugation bei 15 000 g abdekantiert und das resuspendierte Pellet in 1 % Triton X100 inkubiert, wodurch die membrangebundene Fraktion erhalten wurde. Sämliche Fraktionen wurden auf β-Lactamase-Aktivität gemäß Plückthun, A. und Knowles, J.R.: "The consequences of stepwise deletions from the signal-processing site for ß-lactamase", J.Biol. Chem. 262 (1987), 3951-3957 getestet, um die Effizienz des Fraktionierungsschrittes zu erfahren. Die tritonlösliche Fraktion wurde mit PBS 100-fach verdünnt, bevor sie Affinitätssäulen zugegeben wurde. Zur Affinitäts-Chromatographie wurde Hühnerlysozym (Boehringer, Mannheim, FRG) mit Cyanogenbromid-aktivierter Sepharose (Pharmacia) gemäß den Anweisungen des Herstellers gekoppelt. Die Lysozym-Sepharose wurde 20 min bei Raumtemperatur mit den Extrakten inkubiert und in Säulen gegossen, die nachfolgend mit 10 Bad-Volumina von PBS, 1M NaCl bzw. 0,5 M NaCl in 0,1 M NaHCO₃ bei pH 8,3 gewaschen wurden, bevor sie mit 0,05 M Diethylamin eluiert wurden. Sämtliche Fraktionen wurden mit Trichloressigsäure (Endkonzentration 20 %) präzipitiert und in SDS Polyacrylamidgelen aufgetrennt (Laemmli, U.K.: "Cleavage of structural proteins during the assembly of the head of the bacteriophage T4", Nature (1970), 227, 680-685). Western Blots wurden gemäß Towbin, H Steahelin, T. and Gordon, I.: "Electrophoretic transfer of protein from polyacrylamide gels to nitrocellulose sheets: procedures and some applications", Proc. Natl. Acad. Sci. USA (1979), 76, 4350-4354, durchgeführt, indem zweite Antikörper, die an Meerrettich-Peroxidase gekoppelt waren, mit Diaminobenziden als Substrat verwendet wurden.
- Fig. 3:: Gelelektrophorese von zirkulärem einzelsträngigen pSEX

Spur 1: fd, Spur 2: Kontroll-Phagemid pUHE31-1 mit fd,
Spur 3: pSEX1 mit fd.

DNA von fd Virionen und verpackte Phagemid-Partikel wurden auf 0,8 % Agarosegele in lxTBE gemäß Sambrook, J., Fritsch, E.F. und Maniatis, T. in "Molecular Cloning: A Laboratory Manual, "2. Auflage, herausgegeben in Cold Spring Harbor Laboratory (1989) gegeben und mit Ethidiumbromid angefärbt.

### Durchführung:

Zur Herstellung verpackter Phagemide wurden pSEX1 enthaltende E. coli JM101. auf M9 Minimalmedium ausplattiert und 30 h bei 37°C inkubiert. 2 ml des gleichen Mediums wurden mit einer der Kolonien angeimpft und bei 37°C unter kräftiger Belüftung inkubiert, bis eine optische Dichte von etwa 0,2 bei 600 nm erreicht wurde. 0,5 ml LB-Medium und ein 10-facher Überschuß des Phagen fd wurden dann der Kultur zugegeben und diese weitere 3 h bei 37°C inkubiert. Nachdem zweimal vorsichtig bei 15 000 g 5 min lang bei Raumtemperatur zentrifugiert worden war, wurde der Überstand auf eine Endkonzentration von 4 % Polyethylenglykol (Serva PEG 6000) und 0,5 M NaCL eingestellt und über Nacht bei 4°C stehen gelassen. Die Phagemide wurden durch 20 minütige Zentrifugation bei Raumtemperatur und 15 000 g sedimentiert und in 200 µl Tris-EDTA-Puffer, pH 7,5 suspendiert. Phagemid DNA wurde durch zehnminütiges Schütteln mit einem Volumen Phenol, gefolgt von einer Behandlung mit Chloroform-Isopropanol und Präzipitation mit Isopropanol präpariert (Sambrook, J., Fritsch, E.F. and Maniatis, T., in "Molecular Cloning: A Laboratory Manual", 2. Auflage, herausgegeben in Cold Spring Harbor Laboratory (1989)).

**TABELLE**

| Spezifische Anreicherung von verpackten Phagemiden an einer Antigen-Affinitätssäule | | | | | | | | | |
|---|---|---|---|---|---|---|---|---|---|
| | Gesamtes Volumen (ml) | Plattiertes Volumen (µl) | Amp^{r}Kolonien Zahl | Gesamt | plattiertes Volumen (µl) | Gesamt pfu Zahl | Gesamt | Überschuß pfu/Amp | Anreicherungsfaktor |
| Aufgetragen | 10 | 10⁻³ | 51 | 5,1x10⁸ | 10⁻⁶ | 45 | 4,5x10¹¹ | 882 | -- |
| Eluiert | 1,1 | 10⁻¹ | 26 | 2,9x10⁵ | 10⁻² | 19 | 2,1x10⁶ | 7,3 | 121 |

## Patentansprüche

1. Verwendung eines phagemids umfassend DNA kodierend für ein Fusionsprotein aus einem Antikörper und einem Coliphagen pIII-Protein, wobei das Coliphagen pIII-Protein die Amino-terminale Domäne und die Carboxyterminale Domäne eines Coliphagen pIII-Proteins umfaßt, in einer substractiven Selections - methode unter Verwendung von normalen und neoplastischen Zellen zur Identifixierung von Tumor-assoziierten Antigenen.

2. Verwendung nach Anspruch 1, wobei der Antikörper ein Einzelketten-Antikörper ist.

## Claims

1. Use of a phagemid comprising DNA coding for an antibody-coliphage pIII fusion protein wherein the coliphage pIII protein comprises the amino-terminal domain and the carboxy terminal domain of a coliphage pIII protein, in a subtractive selection method using normal and neoplastic cells to identify tumour associated antigens.

2. A use according to claim 1, **characterised in that** said antibody is a single-chain antibody.

## Revendications

1. Utilisation d'un phagemide comprenant de l'ADN de codage d'une protéine de fusion composée d'un anticorps et d'une protéine pIII de coliphage, la protéine pIII de coliphage comprenant les domaines terminaux amino et les domaines terminaux carboxy d'une protéine pIII de coliphage, dans une méthode de sélection soustractive à l'aide de cellules normales et néoplastiques pour l'identification d'antigènes associés à une tumeur.

2. Utilisation selon la revendication 1, l'anticorps étant un anticorps à une seule chaîne.
